# FASCICULE DE BREVET EUROPEEN

(11) **EP 1 806 979 B1**
(45) Date de publication et mention de la délivrance du brevet: **09.06.2010**
(21) Numéro de dépôt: 05815293.5
(22) Date de dépôt: 02.11.2005
(51) Int. Cl.: A23L 1/23, C07D 307/32, C12P 17/04

(54) **GAMMA-UNDECENOLACTONE, PROCEDE DE PREPARATION ET UTILISATIONS EN COSMETIQUES OU COMME ADDITIF ALIMENTAIRE**
GAMMA-UNDECENOLACTON, VERFAHREN ZU SEINER HERSTELLUNG UND SEINE VERWENDUNG FÜR KOSMETIKARTIKEL UND ALS NAHRUNGSZUSATZ
GAMMA-UNDECENOLACTONE, METHOD FOR THE PREPARATION AND USE THEREOF FOR COSMETICS AND IN THE FORM OF FOOD ADDITIVES

(30) Priorité: 03.11.2004 FR 0411721
(43) Date de publication de la demande: 18.07.2007
(73) Titulaire: V. Mane Fils, 06620 Bar sur Loup (FR)
(72) Inventeur: ZUCCA, Joseph, F-06130 Grasse (FR); MANE, Jean, F-06130 Grasse (FR)
(74) Mandataire: Cabinet Plasseraud
(86) Numéro de dépôt international: PCT/FR2005/002729
(87) Numéro de publication internationale: WO 2006/048550

(56) Documents cités:
- EP-A- 0 258 993
- EP-A- 0 519 481
- DE-A1- 4 126 997
- US-A- 4 560 656
- US-A- 4 950 607
- US-A- 5 457 036
- WILSON, STEPHEN R. ET AL: "Silicon-mediated skipped diene synthesis. Application to the melon fly pheromone" JOURNAL OF ORGANIC CHEMISTRY , 53(20), 4682-93 CODEN: JOCEAH; ISSN: 0022-3263, 1988, XP002334828

## Description

La présente invention a pour objet une nouvelle lactone, la gamma-undécénolactone ; sa synthèse par voie biologique, en particulier la synthèse stéréosélective de chacun de ses isomères (R) ou (S) ; et ses utilisations, notamment dans le secteur alimentaire et de la parfumerie.

Les produits « naturels » sont de plus en plus prisés par le grand public, et de ce fait les industries mettant en oeuvre des composés aromatiques ou odorants portent leurs efforts dans la mise au point de substances et de préparations aromatisantes « naturelles ». Seules les substances ayant été identifiées dans la nature peuvent prétendre à ce label ; elles sont donc actuellement produites soit à partir de plantes, soit à partir de microorganismes ; ces derniers sont de plus en plus employés, des procédés biotechnologiques permettant maintenant de synthétiser des molécules naturelles à des coûts raisonnables. C'est le cas des gamma-lactones.

Les gamma-lactones sont des molécules aromatiques constitutives de l'arôme et de la saveur de nombreux produits naturels. Par exemple, la gamma-heptalactone est connue pour son arôme et son goût de noisette ou de caramel, la gamma-nonalactone a un arôme grays, crémeux, ou de noix de coco ; la gamma-décalactone et la gamma-undécalactone ont un arôme et un goût de pêche ou d'abricot.

Les gamma-lactones existent à l'état naturel, sous leurs deux formes énantiomériques (R) et (S), l'énantiomère (R) étant toutefois prédominant.

Les gamma-lactones peuvent être produites par voie synthétique, ou par biosynthèse au moyen de microorganismes. Ainsi, EP 371 568 décrit un procédé de production de gamma-lactones au moyen de micro-organismes acceptables pour faire des produits alimentaires, tels que notamment *Saccharomyces cerevisiae, Debaromyces hansenii* ou *Candida boidinii.*

US 5,112,803 indique que la gamma-octalactone, et en particulier ses isomères optiques (R) et (S), est utile pour former des arômes et des saveurs de beurre, et décrit un procédé pour augmenter l'arôme ou la saveur de matières consommables par ajout de quantités significatives de gamma-octalactones optiquement actives, et d'un mélange de différents composés qui sont des sous-produits du procédé biologique d'obtention des gamma-octalactones décrit dans ce brevet. Le procédé décrit dans US 5,112,803 indique qu'à partir d'acide caprylique, il est possible d'obtenir les deux isomères (R) et (S) de la gamma-octalactone par biosynthèse à l'aide des souches du genre *Syncephalastratum sp.* ou *Mortierella sp. ;* toutefois ce procédé n'est pas énantiosélectif.

L'intérêt des gamma-lactones dans l'industrie aromatique alimentaire et dans l'industrie de la parfumerie est important, et l'obtention de produits ayant des nuances organoleptiques différentes, représente un véritable enjeu industriel.

L'objet de la présente invention est de proposer une nouvelle gamma-lactone, la gamma-undécénolactone, qui présente des propriétés aromatiques et gustatives comparables à celles des gamma-lactones connues, mais différentes de celles-ci, en particulier des arômes et saveurs d'ananas et de fruits de la passion.

Il est connu que la chiralité des molécules volatiles peut induire des différences au niveau de la perception olfactive, et que les isomères optiques des gamma-lactones n'ont pas tous les mêmes notes organoleptiques : il y a donc un intérêt important à obtenir un isomère optique particulier de gamma-lactone, en particulier si cette obtention se fait suivant un procédé au moins aussi efficace, voire plus efficace que dans l'art antérieur et à un prix compétitif.

Un autre objet de l'invention est donc de proposer un procédé de synthèse par voie biologique de la gamma-undécénolactone efficace et économique, et en particulier un procédé de synthèse stéréosélective de chacun des isomères optiques (R) et (S) de la gamma-undécénolactone.

La gamma undécénolactone conforme à l'invention répond à la formule (I) : 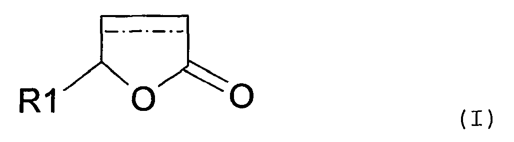 dans laquelle le cycle lactonique peut porter une insaturation entre le carbone N°2 et le carbone N°3 et R1 est un groupe alcènyle en C₇ portant une seule insaturation alcènique en C10-C11, ou un groupe alcènyle en C₇ comprenant plusieurs insaturations dont une insaturation alcènique en C10-C11 et au moins une autre insaturation alcènique sur un carbone autre que C7, ledit groupe R1 étant éventuellement substitué.

Suivant un mode de réalisation préféré de l'invention, R1 comprend une seule insaturation.

Suivant un autre mode de réalisation de l'invention, R1 comprend plusieurs insaturations, dont une insaturation alcénique en C10 et au moins une autre insaturation alcénique sur un carbone autre que C7.

Suivant un mode de réalisation particulier de l'invention, R1 est un groupe alcénique ayant 7 atomes de carbones, éventuellement substitué, portant une seule insaturation située en C10-C11.

Suivant un autre mode de réalisation préféré de l'invention, R1 n'est pas substitué par un atome d'halogène, en particulier le brome ou le chlore.

Suivant un mode de réalisation préféré de l'invention, R1 est un groupe C₇H₁₃, de préférence le groupe CH₂=CH-CH₂-CH₂-CH₂-CH₂-CH₂-, et le cycle lactonique est saturé.

Par alcényle substitué on entend un alcényle dont au moins un carbone porte au moins un groupe substituant. Par groupe substituant on entend notamment un groupe hydroxyle, un groupe céto, un groupe thiol, un groupe alkyle ou un groupe alcényle.

La gamma-undécénolactone selon l'invention présente un carbone asymétrique en position 4 et peut être de configuration (R) ou (S).

L'invention concerne aussi bien la (R) gamma-undécénolactone que la (S) gamma-undécénolactone, et le mélange, en quelques proportions que ce soit, de ces deux isomères optiques, en particulier le mélange racémique.

L'invention concerne également la préparation de la gamma-undécénolactone de formule (I) par voie biologique et en particulier sa biosynthèse par voie microbienne, à partir d'au moins un substrat, notamment l'acide undécylénique ou l'un de ses esters, de préférence méthylique ou éthylique, à l'aide d'une culture microbienne d'une souche choisie parmi celles permettant une hydroxylation du substrat, en particulier en C4.

Cette préparation comprend les étapes suivantes :
a) sélection d'une souche appropriée,
b) culture de ladite souche dans un milieu de culture adéquat, ladite culture étant éventuellement précédée d'une étape de préculture de la souche,
c) ajout d'un substrat susceptible d'être transformé en gamma-undécénolactone de formule (I),
d) bioconversion du substrat en gamma-undécénolactone de formule (I),
e) récupération de la gamma-undécénolactone de formule (I) ainsi produite.

Les souches microbiennes appropriées, visées à l'étape a) pour la biosynthèse de la gamma-undécénolactone selon l'invention sont celles permettant une hydroxylation spécifique du substrat, en particulier en C4.

Lorsque le produit résultant de la biosynthèse est destiné à l'industrie alimentaire, les souches de grade alimentaire sont bien entendu préférées. Parmi les souches permettant une hydroxylation stéréosélective, on peut citer notamment les souches du genre *Aspergillus sp., Penicillium sp., Mucor sp., Mortierella sp.* Ces souches appartenant toutes à la classe 1 de microorganismes, et certaines étant alimentaires, leur utilisation ne pose pas de problème particulier tant pour la production industrielle de la lactone que pour son utilisation éventuelle en alimentaire.

Suivant un mode de réalisation particulier de l'invention, la souche utilisée est du genre *Aspergillus sp.,* de préférence *Aspergillus oryzae* dont on peut citer les souches de collections suivantes :
*Aspergillus oryzae DSMZ* 1861, *Aspergillus oryzae* DSMZ 1864, *Aspergillus oryzae* DSMZ 1147, *Aspergillus oryzae* DSMZ 63303, *Aspergillus oryzae* CBS 570.65, *Aspergillus oryzae* CBS 819.72, *Aspergillus oryzae* CBS 110.27, *Aspergillus oryzae* VMF 88093.

Parmi elles, *Aspergillus oryzae* DSMZ 1861 et *Aspergillus oryzae* CBS 110.27 sont préférées.

Suivant un autre mode de réalisation particulier, la souche utilisée est du genre *Mortierella sp*. dont on peut citer les espèces de collections suivantes :
*Mortierella isabellina* DSMZ 1414, *Mortierella isabellina* CBS 100559, *Mortierella isabellina* CBS 221.29, *Mortierella isabellina* CBS 194.28, *Mortierella isabellina* CBS 208.32, *Mortierella isabellina* CBS 224.35, , *Mortierella isabellina* CBS 560.63, *Mortierella isabellina* CBS 167.80, *Mortierella isabellina* CBS 493.83, , *Mortierella isabellina* CBS 309.93, *Mortierella isabellina* CBS 250.95, *Mortierella isabellina* CBS 109075, *Mortierella ramanniana* CBS 112.08, *Mortierella ramanniana* CBS 219.47, *Mortierella ramanniana* CBS 243.58, *Mortierella ramanniana* CBS 478.63, *Mortierella ramanniana* CBS 852.72, *Mortierella ramanniana* CBS 366.95, *Mortierella ramanniana* CBS 101226.
   Parmi elles, les souches de *Mortierella isabellina* seront préférées, en particulier *Mortierella isabellina* DSMZ 1414, *Mortierella isabellina* CBS 100559 et *Mortierella isabellina* CBS 221.29.

En effet, les inventeurs ont remarqué que, de manière surprenante et inattendue, l'utilisation d'une souche du genre *Aspergillus sp.* conduit à la production sélective de (R) gamma-undécénolactone, et que l'utilisation d'une souche du genre *Mortierella sp.* conduit à la production sélective de (S) gamma-undécénolactone.

Suivant un mode de réalisation de l'invention, sont exclues de l'invention les souches Yarrowia lipolytica, car elles ne sont pas susceptibles de provoquer une hydroxylation en C4. Avantageusement, toutes les souches qui ne sont pas susceptibles de produire, de manière spécifique, et de manière stéréosélective, une hydroxylation en C4, sont exclues de la présente invention.

Sans vouloir être lié par une quelconque théorie, il est envisageable que les conditions de culture des souches pourraient avoir une importance dans la stéréosélectivité remarquée, ainsi que dans l'aspect quantitatif de la bioconversion.

La culture visée à l'étape b) du procédé selon l'invention, comprend la réalisation d'une culture, de préférence semi-concentrée, des souches, par exemple par amplification cellulaire, dans un milieu de culture approprié. Cette culture peut être précédée par une pré-culture des souches dans un premier milieu de culture plus adapté aux premières étapes de multiplication de la souche.

Les conditions de culture mises en oeuvre dans le procédé stéréosélectif de l'invention doivent être telles qu'elles conduisent à la production d'un mycélium qui présente des boursouflures remplies d'inclusions (de péroxysomes en particulier). Selon le mode de réalisation préféré de l'invention, la culture cellulaire réalisée présente un mycélium « compoteux » composé de filaments cloisonnés sans conidiospores et présentant des structures renflées, remplies de ces inclusions (péroxysomes). Les conditions de culture doivent être en effet particulièrement adaptées pour éviter la sporulation du mycélium.

Par ailleurs, les Inventeurs ont pu constater que l'état physiologique du mycélium, obtenu notamment du fait de la mise en oeuvre des conditions de cultures décrites dans la présente demande, (mycélium cloisonné comportant des boursouflures et renflements remplies d'inclusions, notamment de péroxysomes) pourrait avoir une influence importante sur le rendement de la réaction et permettrait d'obtenir des rendements supérieurs à ceux de l'art antérieur. L'état physiologique du mycélium pourrait également avoir une influence sur la stéréosélectivité de la réaction.

Ainsi, suivant un mode de réalisation préféré de l'invention, l'étape) du procédé de l'invention est une étape de culture de la souche dans un milieu de culture approprié permettant b l'obtention d'un mycélium cloisonné comportant des boursouflures et renflements remplis d'inclusions, notamment de péroxysomes. Avantageusement, le milieu de culture utilisé selon l'invention ne contient pas de peptone. Préférentiellement, le milieu de culture utilisé selon l'invention comprend du malt et/ou de l'extrait de levure. Suivant un mode de réalisation préféré, le mycélium utilisé pour l'étape c) est concentré. De préférence, la concentration du mycélium utilisé pour l'étape c) est comprise entre 5 et 15 g/l, de préférence 6 à 12 g/l, très préférentiellement 7 à 10 g/l.

Il a été particulièrement remarqué que la production de (S) gammalactone par la souche Mortierella est particulièrement favorisée, en terme de stéréosélectivité et en terme de rendement, par la mise en oeuvre d'un mycélium boursouflé et rempli d'inclusions tel que décrit ci-dessus ; en effet, l'utilisation d'un tel mycelium permettrait l'obtention d'un produit de réaction ayant un pouvoir rotatoire supérieur, en valeur absolue, à ceux de l'art antérieur ; d'autre part, le rendement obtenu par le procédé selon l'invention, et en particulier par la mise en oeuvre d'un mycélium boursouflé et rempli d'inclusions tel que décrit ci-dessus, permet l'obtention de rendements supérieurs à ceux de l'art antérieur.

L'étape c) du procédé consiste à ajouter le substrat à la culture cellulaire. Selon l'invention, la synthèse par voie biologique de gamma-undécénolactone fait intervenir tout substrat approprié et de préférence l'acide undécylénique ou l'un de ses esters. Parmi les autres substrats préférés, on peut citer tous les dérivés substitués de l'acide undécylénique ou de ses esters. L'acide undécylénique, l'ester méthylique et l'ester éthylique de l'acide undécylénique sont des substrats particulièrement préférés. Il va de soi que le substrat peut être tout substrat approprié, ou un mélange de différents substrats appropriés, en particulier un mélange d'acide undécylénique et d'un ou plusieurs de ses esters.

Suivant un mode de réalisation avantageux de l'invention, le substrat est ajouté au mycélium suivant un procédé batch ou fed-batch. Suivant un mode de réalisation préféré, l'acide undécylénique est ajouté en mélange avec un auxiliaire, par exemple une huile, notamment toute huile classique alimentaire telle que soja, maïs, tournesol, ou autre ou triglycérides de synthèse d'acides gras à chaînes courtes tels que miglyol, de préférence l'huile de tournesol hydrogénée ou riche en acide oléique, préalablement à sa mise en contact avec le mycélium. La présence de l'auxiliaire permet notamment de diminuer fortement l'effet corrosif ou toxique du substrat, notamment de l'acide undécylénique. Suivant un mode de réalisation de l'invention, la synthèse selon l'invention en utilisant la souche Mortierella isabellina s'effectue dans un milieu exempt d'huile minérale.

Avantageusement, le substrat est ajouté dans des concentrations de 0.3 à 2.5g/l/h. Avantageusement, la quantité d'huile, de préférence d'huile végétale, mélangée au substrat est de 100 à 500 g/l, de préférence 150 à 300 g/l.

Une source de sucre, de préférence de glucose, est également ajoutée au milieu, en même temps que le substrat de manière à assurer la couverture des besoins énergétiques des cellules. Avantageusement, la concentration en glucose ajoutée est de 0.3 à 0.4 g/l/h

Le pH peut être régulé, suivant les besoins, pendant l'addition du substrat et pendant toute la durée de la bioconversion qui va suivre, au moyen de l'addition de toute base appropriée. Le pH doit être compris entre 4,5 et 8,5, de préférence entre 5,5 et 8 et de préférence entre 6 et 7,5.

La température est de préférence maintenue entre 27 et 30 °C, pendant la bioconversion. La durée de la bioconversion peut être de 30 à 120 heures, de préférence de 48 à 72 heures.

La bioconversion du substrat en gamma-undécénolactone visée à l'étape d) du procédé de l'invention, est une étape de lactonisation précédée par une réaction d'hydroxylation en C4 du substrat, réalisée par la souche. Pour que cette hydroxylation puisse s'effectuer, une source d'oxygène est requise. Cette source d'oxygène est de préférence un gaz contenant de l'oxygène, très préférentiellement l'air ou l'oxygène. Le gaz est dissous en relativement grande quantité dans le milieu réactionnel.

Suivant un mode de réalisation préféré, et comme il est connu dans l'état de la technique, des agents anti-mousse, notamment des huiles siliconées ou des polymères de polyéthylène glycol estérifiés par des acides gras, sont utilisés pour contrôler la mousse susceptible de se former lors de la bioconversion.

Une fois la bioconversion, c'est-à-dire l'hydroxylation spécifique et stéréoselective en C4, suivie de la lactonisation, effectuée, l'étape e) du procédé consiste à récupérer la gamma-undécénolactone par extraction, l'extraction de la gamma-undécénolactone s'effectuant par tout moyen approprié. Avantageusement, l'extraction de la gamma-undécénolactone est effectuée par hydrodistillation, éventuellement suivie d'une estérification destinée à l'élimination ultérieure du substrat n'ayant pas réagi.

Alternativement, l'extraction de la gamma-undécénolactone est effectuée par extraction par solvant (cyclohexane, acétate d'éthyle), après acidification du milieu.

Suivant une variante du procédé selon l'invention, l'étape e) du procédé n'est pas réalisée, et à la place, on effectue une étape e') qui consiste à poursuivre le procédé à la fin de l'étape d) par une réduction in situ de la gamma-undécénolactone obtenue, avant extraction, pour obtenir par exemple la gamma-undécalactone correspondante. Suivant un mode de réalisation particulier, la réduction peut être stoppée pour obtenir une gamma-undécénolactone ayant moins d'insaturations que celle issue de la bioconversion de l'étape d) . _ Selon cet autre mode de réalisation, le procédé selon l'invention est poursuivi à l'issue de l'étape d), en stoppant la régulation pH du fermenteur, et en ajoutant une levure sèche active qui peut être une levure boulangère, une levure de vinification ou une levure de brasserie et une source de sucre, notamment de glucose, dans le réacteur. Quand le pH atteint la valeur de 5,5, il est régulé à 5,5 avec une base appropriée, par exemple de la soude. On laisse incuber pour que la réduction se fasse, de préférence pendant un temps de 12 à 24 heures, puis on extrait la gamma-undécalactone dans les conditions décrites à l'étape e). Suivant une autre variante, la gamma-undécénolactone peut être réduite en gamma-undécalactone par une culture fraîche d'un microorganisme réducteur ou du moins placé dans des conditions réductrices, par exemple, *Saccharomyces cerevisiae* ou *Pichia etchelsii* ou *Pichia pastoris* ou *Hansenula polymorpha* ou *Bacillus subtilis* ou *Lactobacillus brevis.*

Suivant un mode de réalisation particulier de l'invention, la réduction de l'étape e') aboutit à la production de gamma-undécalactone et cette gamma-undécalactone répond à la formule : 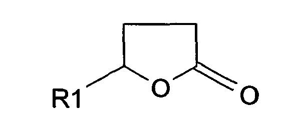 dans laquelle R1 est un groupe alkyle en C₇, éventuellement substitué. Par alkyle substitué on entend un alkyle dont au moins un carbone porte au moins un groupe substituant. Par groupe substituant on entend notamment un groupe hydroxyle, un groupe céto, un groupe thiol, un groupe alkyle ou un groupe alcényle.

Suivant un mode de réalisation préféré de l'invention, R1 est le groupe C₇H₁₃, très préférentiellement CH₂=CH-CH₂-CH₂-CH₂-CH₂-CH₂- .

La gamma undécalactone obtenue selon ce mode de réalisation particulier présente en position 4 un carbone asymétrique de même configuration que celui de la molécule de gamma undécénolactone dont elle dérive, la réaction de réduction ne modifiant pas la stéréoisomérie de la molécule.

L'étape e') peut être également réalisée et permet d'obtenir une undécénolactone plus saturée (R) ou (S) ou une gamma-undécalactone (R) ou (S), suivant la stéréochimie de la gamma undécénolactone obtenue à l'étape d).

Ces lactones saturées, la (R) gamma-undécalactone et la (S) gamma-undécalactone peuvent être employées dans les mêmes applications que leurs homologues insaturées : ces lactones ont des propriétés odorantes et gustatives telles qu'elles peuvent être utilisées dans toutes les applications de parfumerie et d'aromatique alimentaire, en particulier pour la fabrication de parfums, de matières odorantes, de compositions cosmétiques ou alimentaires, ou comme additif alimentaire.

Au sens de la présente invention, le terme parfumerie désigne non seulement la parfumerie au sens habituel du terme, mais également les autres domaines dans lesquels l'odeur des produits est importante. Il peut s'agir de compositions de parfumerie au sens habituel du terme, telles que bases et concentrés parfumants, eaux de Cologne, eaux de toilette, parfums et produits similaires ; de compositions topiques - en particulier cosmétiques - telles que crèmes pour le visage et le corps, poudres de talc, huiles pour cheveux, shampoings, lotions capillaires, sels et huiles de bain, gels de douche et de bain, savons de toilette, anti-transpirants et désodorisants corporels, lotions et crèmes de rasage, savons, crèmes, dentifrices, bains de bouche, pommades, et produits similaires ; et de produits d'entretien, tels qu'assouplissants, détergents, lessives, désodorisants d'ambiance, et produits similaires.

Le terme odorant est utilisé pour désigner un composé qui exhale une odeur.

Par aromatique alimentaire, on entend toute utilisation des composés de l'invention pour l'aromatisation de toute denrée alimentaire liquide ou solide, humaine ou animale notamment des boissons, des produits laitiers, des crèmes glacées.

La gamma undécénolactone, (R) ou (S), ou un mélange de (R) et (S), ainsi que la gamma-undécalactone (R) ou (S), ou un mélange de (R) et (S) sont utilisables en compositions parfumantes pour contribuer à donner des notes exotiques, florales ou fruitées, ce qui a conduit la Demandresse à déposer le nom de marque « Tropicalone ® » donné à la gamma undécénolactone. Selon les applications, on utilisera l'énantiomère (S) ou l'énantiomère (R) ou encore un mélange des 2 énantiomères dans des proportions déterminées par l'homme de l'art.

De préférence, les produits selon l'invention sont utilisés dans des quantités comprises entre 0,0025% et 10% en poids par rapport au poids total de la composition dans laquelle ils sont présents. Ils peuvent entrer dans la composition de solides ou de liquides et notamment dans la composition de gels, crèmes, pommades et/ou sprays.

Les produits selon l'invention peuvent également être utilisés dans une composition elle-même odorante, ou dans une composition dans laquelle l'agent odorant est utilisé pour masquer ou neutraliser certaines odeurs.

D'autres caractéristiques et avantages de la présente invention apparaîtront clairement à la lecture des exemples donnés ci-après qui viennent illustrer l'invention sans toutefois la limiter.

### Exemple 1, Etape a : - Sélection des souches

Toutes les souches de la collection sont d'abord ensemencées sur milieu gélosé MGY et incubées pendant 72 h à 27°C ; par la suite, ces souches sont ensemencées dans des Erlenmeyers de 1 Litre contenant 100 ml de milieu au malt 1x et incubées pendant 24 h à 27°C. Le substrat, l'acide undécylénique, est alors ajouté dans le milieu de culture (5 g/l en 10 doses) et la culture est maintenue pendant encore 48 h à 120 h à 27°C.

Après olfaction et analyses de la concentration en gamma undécénolactone dans les milieux, les souches les plus intéressantes sont retenues ; cela a été le cas pour les souches de Mortierella isabellina CBS 100559, Mortierella isabellina CBS 221.29, Aspergillus oryzae DSMZ 1861 et Aspergillus oryzae CBS 110.27 qui ont par la suite été utilisées pour les essais d'optimisation en fermenteurs.

### Exemple 1, Etape b : - Préparation des cultures cellulaires

On ensemence la souche Mortierella isabellina CBS 100559 ou Mortierella isabellina CBS 221.29 ou Aspergillus oryzae DSMZ 1861 ou Aspergillus oryzae CBS 110.27 origine = tube congelé à - 80°C) sur gélose MGY, et on incube à 27°C pendant 30 heures.

On ensemence la préculture précédente dans 5 1 de milieu au malt 1x en fermenteur de 6 1 :

| | |
|---|---|
| *Extrait de malt :* | *165 g* |
| *Extrait de levure :* | *25 g* |
| *H₂O q.s.p. :* | *5 l* |
| *pH :* | *6,5* |

### Mortierella isabellina

On incube à 27°C, 500 rpm, 3,5 1/1/h d'air, pH libre, pendant 30 heures.

### Aspergillus oryzae

On incube à 20°C, 500 rpm, 0,05 vvm d'air, pH libre, pendant 30 h puis à 25°C, 500 rpm, 0,05 vvm d'air, pH libre, pendant 24 heures. On doit obtenir dans les deux cas un mycélium contenant beaucoup de boursouflures grosses et pleines d'inclusions (dont des péroxysomes).

On prépare ensuite 125 1 de milieu au malt 1,5x dans un fermenteur de 300 1 :

| | |
|---|---|
| *Extrait de malt :* | *6,188 kg* |
| *Extrait de levure :* | *0,938 kg* |
| *H₂O q.s.p. :* | *125 l* |

On stérilise le milieu 40 minutes à 121°C. Le fermenteur et ses utilités sont stériles et sous pression. La température est stable et régulée à 27°C. On chasse la pression et on maintient un débit d'air de 3,5 1/1/h, soit environ 0,6 m³/h. On raccorde stérilement la base (NaOH 10 N), l'acide (H₃PO₄ 85%) et l'antimousse et le fermenteur de 6 1 qui sert d'inoculum. On ajuste la vitesse d'agitation à 325 rpm, on amorce l'antimousse, puis on ensemence l'inoculum (5 1), pH libre. On maintient la vitesse d'agitation à 325 rpm et on augmente l'aération à 2,2 m³/h (0,3 vvm). On laisse pousser pendant 24 heures de façon à avoir environ 10 g/l de poids sec de mycélium : ce mycélium doit être « compoteux » et constitué de filaments comportant de nombreux renflements et boursouflures, sans spores.

### Exemple 2, Etapes c et d : Conversion de l'acide undécylénique par les souches de Mortierella sp.

Une fois la quantité et la qualité de mycélium atteintes, on distribue l'acide undécylénique au débit de 0,3 g/l/h pendant 6 H puis au débit de 0,53 g/l/h pendant 72 h : soit un total de 40 g/l. Cet acide undécylénique est distribué en mélange avec de l'huile de tournesol hydrogénée (1/4 acide-3/4 huile) ; cette huile est donc distribuée aux débits de 0,9 g/l/h puis de 1,53 g/l/h. On distribue parallèlement du glucose en continu au débit de 0,36 g/l/h pendant 72 h. On régule le pH à 7,5 pendant toute la durée de la fermentation avec NaOH 5 N. On augmente la vitesse à 505 rpm et on aère au débit de 1 vvm, soit 12 m³/h. On poursuit la conversion pendant 72 heures.

On obtient une production de 6,5 g/l de gamma undécénolactone dont la stéréoisomérie est(S).

### Exemple 3, Etapes c et d : conversion de l'acide undécylénique par les souches d'Aspergillus sp.

Une fois la quantité et la qualité de mycélium atteintes, on distribue l'acide undécylénique au débit de 0,3 g/l/h pendant 6 H puis de 0,53 g/l/h pendant 72 h : soit un total de 40 g/l. Cet acide undécylénique est distribué en mélange avec de l'huile de tournesol hydrogénée (1/4 acide-3/4 huile). On distribue parallèlement du glucose en continu au débit de 0,36 g/l/h pendant 72 h. On régule le pH à 6,5 pendant toute la durée de la fermentation avec NaOH 5 N. On aère au débit de 0,5 vvm, soit 6 m³/h. On poursuit la conversion pendant 80 heures. On obtient une production de 0,5 g/l de gamma undécénolactone dont la stéréoisomérie est (R)

### Exemple 4, Etape e : extraction - purification

On acidifie à pH 1,5 avec 3 1 d'acide phosphorique à 85%. On chauffe à plus de 100°C pendant 30 minutes pour que la lactone se présente essentiellement sous sa forme cyclisée et non pas sous sa forme ouverte d'hydroxyacide. On dose la lactone, on ajoute du solvant d'extraction (cyclohexane de préférence), on agite 1 heure à température ambiante. On centrifuge et on récupère la phase organique. On dose la lactone. On concentre le solvant et on obtient ainsi un "brut" huileux. On distille sous vide. On obtient la lactone "dérésinée" et une huile épuisée. On purifie ensuite en fractionnant la lactone sous vide. On obtient un produit pur à > 99%, qui est soit de la gamma undécénolactone (> 99% S) si on a utilisé une souche de *Mortierella sp.,* soit de la gamma undécénolactone (> 99% R) si on a utilisé une souche d'*Aspergillus sp.*

### Exemple 5, Etape e' : réduction de la gamma-undécénolactone en gamma-undécalactone

Au lieu d'arrêter la réaction, après la bioconversion de l'exemple 3, on procède comme suit :
On stoppe la régulation de pH du fermenteur. On ajoute dans le milieu : 200 g/l de levure sèche active de levure boulangère du commerce (soit 30 kg de levure) et 100 g/l de glucose (soit 15 kg de glucose sec ou 30 kg de sirop de glucose à 50%). A partir du moment où le pH atteint la valeur de 5,5, on le régule à pH 5,5 avec une base NaOH 5 N. On incube pendant 12 à 24 H à 30°C, 325 rpm, air 0,5 vvm. On obtient de la gamma-undécalactone (S) ou de la gamma-undécalactone (R) selon le cas. On effectue ensuite l'extraction et la purification conformément à l'exemple 4 ci-dessus.

### Exemple 6 : évaluation de la gamma-undécénolactone (S) en parfumerie

La gamma undécénolactone (S) pure à 99% a été testée sur mouillette et en solution (à 5% dans l'éthanol) : la tête est aldéhydée lactonique de type gamma undécalactone aldehyde intreleven (IFF), très puissante. Le fond est ananas chair très naturel, lactonique et très puissant.

Elle a également été testée en formulation où elle apporte une note fruitée marine intéressante, par exemple dans la formule suivante :

| | |
|---|---|
| Alcool phényl éthylique | 150 g |
| calone | 1 g |
| Citronellol | 20 g |
| Dipropylène glycol | 50 g |
| Galaxolide | 200 g |
| Méthyl Dihydro Jasmonate | 150 g |
| Helional | 15 g |
| Indole | 2 g |
| Béta Ionone | 15 g |
| IsoE super | 150 g |
| Lilial | 130 g |
| Linalol | 49 g |
| Melonal | 3 g |
| Methylionantheme | 15 g |
| Gamma undécénolactone (Tropicalone®) | 50 g |

### Exemple 7 : évaluation de la gamma-undécénolactone (S) en aromatique alimentaire

La gamma undécénolactone (S) pure à 99 % a été testée à 10 ppm dans l'eau minérale : elle présente une note boisée et fruitée très différente de celle donnée par toute autre lactone utilisée jusqu'à présent en formulation ; en comparaison à la gamma-décalactone (R), à la delta décalactone (R) et à la delta-dodécalactone (R), au même dosage (10 ppm), elle est plus coco, plus boisée avec des notes ananas et fruit de la passion.

Dans l'eau sucrée (10 ppm), elle présente aussi des notes à caractère laitier, très gras, sucré, doux. Sa puissance à 10 ppm lui confère un intérêt en formulation en raison de son caractère laitier unique, tendance lait concentré.

Testée également à 0.5 ppm et 1 ppm sur un arôme crème : elle ressort meilleure en bouche à 0.5 ppm, en arrondissant la crème et en lui donnant un caractère unique type lait condensé.

### Exemple 8 : évaluation de la gamma-undécalactone (S) en aromatique alimentaire

Comparée pure à 99% sur mouillette à la gamma undécalactone (R), la gamma undécalactone (S) présente des notes moins grasses, plus naturelles et plus « pêche ».

Evaluée à 10 ppm dans l'eau minérale, elle présente un goût de pêche et d'abricot caractéristique.

### Exemple 9 : évaluation de la gamma-undécalactone (S) en formulation en aromatique alimentaire.

Dans un arôme de fruits exotiques, l'ajout de gamma undécénolactone augmente l'appréciation « Exotique », par exemple dans la formule suivante :

| | |
|---|---|
| Rose oxide | 10,4 g |
| Phényle Oxyde | 10,4 g |
| aldéhyde phénylpropylique | 20,7 g |
| Néryle acétate | 0,30 g |
| gamma undécalactone | 1 g |
| gamma nonalactone | 1 g |
| Styrax | 6,2 g |
| Acétyl Méthyl carbinol | 10,4 g |
| Delta Dodécalactone | 10,4 g |
| Gamma décalactone | 10,4 g |
| Acide Caproique | 20,7 g |
| Furanéol | 21,75 g |
| Essence térébenthine | 31,1 g |
| Alcool hexylique | 41,5 g |
| Caryophyllène | 51,8 g |
| para Cymène | 0,2 g |
| Citronellyle propionate | 6,2 g |
| Nérol | 20,7 g |
| Hexyle caproate | 20,7 g |
| Citral 1,3, Cis 3 hexenol | 103,6 g |
| Triacétine | 599,25 g |
| Gamma undécénolactone (Tropicalone®) | 1,5 g |

Même effet dans un arôme de fruit de la passion :

| | |
|---|---|
| Alcool butylique | 1,00, |
| Alcool Hexylique | 1,00 g |
| Hexanol 1 Ethyl 2 | 1,60 g |
| Alcool Caprylique | 2,10 g |
| Damascenone | 3,10 g |
| Butyle Butyrate | 4,70 g |
| Beta Ionone | 21 g |
| Cis 3 Hexenyle Acétate | 52,60 g |
| Heptyle Butyrate | 78,8 g |
| Gamma Décalactone | 79 g |
| Acide caproique | 105 g |
| Butyle isovalérate | 105,10 g |
| Delta Nonalactone | 1,00 g |
| Héxyle caproate | 42 g |
| Essence Orange Brésil | 131,4 g |
| Butyle acétate | 1, 00 g |
| Acide Butyrique | 1,60 g |
| Aldéhyde Benzoique | 52,60 g |
| Ethyle butyrate | 157,7 g |
| Cis 3 Hexenol | 157,7 g |
| Gamma Undécénolactone | 2,5 g |

## Revendications

1. Gamma-undécénolactone répondant à la formule (I): 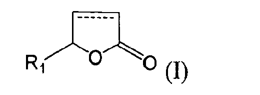 dans laquelle le cycle lactonique peut porter une insaturation entre le carbone N°2 et le carbone N°3 et R1 est un groupe alcènyle en C₇ portant une seule insaturation alcènique en C10-C11, ou un groupe alcènyle en C₇ comprenant plusieurs insaturations dont une insaturation alcènique en C10-C11 et au moins une autre insaturation alcènique sur un carbone autre que C7, ledit groupe R1 étant éventuellement substitué.

2. Gamma-undécénolactone selon la revendication 1, dont le carbone asymétrique en position 4 est de configuration (R).

3. Gamma-undécénolactone selon la revendication 1, dont le carbone asymétrique en position 4 est de configuration (S).

4. Gamma-undécénolactone selon l'une quelconque des revendications 1 à 3 dans laquelle le cycle lactonique ne comporte pas d'insaturation et R1 est un groupe C7H13, de préférence CH2=CH-CH2-CH2-CH2-CH2-CH2-.

5. Procédé de préparation d'une gamma-undécénolactone telle que décrite dans l'une quelconque des revendications 1-4, **caractérisé en ce que** l'on effectue une biosynthèse par voie microbienne de ladite gamma-lactone, à partir d'au moins un substrat, notamment l'acide undécylénique ou l'un de ses esters, de préférence méthylique ou éthylique, à l'aide d'une culture microbienne d'une souche choisie parmi celles permettant une hydroxylation en C4 du substrat.

6. Procédé selon la revendication 5, **caractérisé en ce qu'**il comprend les étapes suivantes :
a) sélection d'une souche appropriée, choisie parmi celles permettant une hydroxylation en C4 du substrat.
b) culture de ladite souche dans un milieu de culture approprié, ladite culture étant éventuellement précédée d'une étape de pré-culture de la souche,
c) ajout d'un substrat susceptible d'être transformé en gamma-undécénolactone de formule (I) telle que définie dans la revendication 1,
d) bioconversion du substrat en gamma-undécénolactone de formule (I),
e) récupération de la gamma-undécénolactone de formule (I) produite.

7. Procédé selon l'une quelconque des revendications 5 ou 6, **caractérisé en ce que** ledit substrat est ajouté à l'étape c) en mélange avec au moins un auxiliaire de fabrication, de préférence choisi parmi les huiles, notamment de l'huile de tournesol hydrogénée ou riche en acide oléique, du mygliol ou du glucose ou un mélange de ces ingrédients.

8. Procédé selon l'une quelconque des revendications 5 à 7, **caractérisé en ce que** l'étape e) est une extraction de la gamma-undécénolactone par hydrodistillation, éventuellement suivie d'une estérification et élimination du substrat n'ayant pas réagi.

9. Procédé selon l'une quelconque des revendications 5 à 7, **caractérisé en ce que** l'étape e) est une extraction par solvant de la gamma-undécénolactone obtenue à l'issue de l'étape d).

10. Procédé selon l'une quelconque des revendications 5 à 7, **caractérisé en ce que** l'étape e) est précédée par une étape e') de réduction in situ de l'undécénolactone obtenue à l'issue de l'étape d).

11. Procédé selon la revendication 10, **caractérisé en ce que** le produit issu de ladite étape e') est une undécalactone.

12. Procédé stéréosélectif de préparation de gamma-undécénolactone selon l'une quelconque des revendications 5 à 10, **caractérisé en ce que** la souche microbienne visée à l'étape a) est choisie parmi les souches du genre *Aspergillus sp.,* de préférence *Aspergillus oryzae* ou parmi les souches du genre *Mortierella sp.,* de préférence *Mortierella isabellina.*

13. Composition de parfumerie **caractérisée en ce qu'**elle comprend une undécénolactone telle que définie dans l'une quelconque des revendications 1 à 4.

14. Composition alimentaire, **caractérisée en ce qu'**elle comprend une undécénolactone telle que définie dans l'une quelconque des revendications 1 à 4.

15. Additif alimentaire, **caractérisée en ce qu'**il comprend une undécénolactone telle que définie dans l'une quelconque des revendications 1 à 4.

16. Utilisation de gamma-undécénolactone telle que définie dans l'une quelconque des revendications 1 à 4 pour la fabrication de parfums, de matières ou compositions odorantes, de compositions cosmétiques, ou comme additif alimentaire odorant et/ou sapide.

17. Utilisation de gamma-undécénolactone telle que définie dans l'une quelconque des revendications 1 à 4 en aromatique alimentaire.

18. Utilisation d'une culture *d'Aspergillus oryzae* pour la biosynthèse de (R) gamma-undécénolactone telle que définie dans la revendication 2.

19. Utilisation d'une culture de *Mortierella isabellina* pour la biosynthèse de (S) gamma-undécénolactone telle que définie dans la revendication 3.

## Claims

1. A gamma-undecenolactone corresponding to formula (I) : 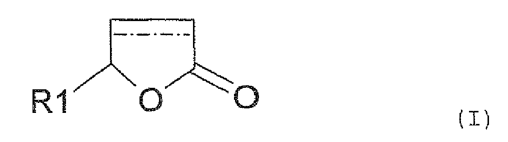 in which the lactone ring can bear an unsaturation between carbon No. 2 and carbon No. 3, and in which R1 is a C₇ alkenyl group bearing a single C₁₀-C₁₁ alkenic unsaturation or a C₇ alkenyl group comprising several unsaturations including a C₁₀-C₁₁ alkenyl unsaturation and at least one other alkenyl unsaturation on a carbon other than C₇, said R1 group being optionally substituted.

2. The gamma-undecenolactone as claimed in claim 1, in which the asymmetric carbon in position 4 is in the (R) configuration.

3. The gamma-undecenolactone as claimed in claim 1, in which the asymmetric carbon in position 4 is in the (S) configuration.

4. The gamma-undecenolactone as claimed in any one of claims 1 to 3, in which the lactone ring does not contain an unsaturation and R1 is a C₇H₁₃ group, preferably CH₂=CH-CH₂-CH₂-CH₂-CH₂-CH₂- .

5. A process for preparing a gamma-undecenolactone as described in any one of claims 1 to 4, **characterized in that** biosynthesis of said gamma-lactone is carried out via the microbial pathway, from at least one substrate, in particular undecylenic acid or one of its esters, preferably methyl or ethyl ester, using a microbial culture of a strain chosen from those that allow hydroxylation of the substrate at C₄.

6. The process as claimed in claim 5, **characterized in that** it comprises the following steps:
a) selecting an appropriate strain, chosen from those that allow hydroxylation of the substrate at C₄,
b) culturing said strain in an appropriate culture medium, said culturing being optionally preceded by a step consisting in preculturing the strain,
c) adding a substrate that can be converted into gamma-undecenolactone of formula (I) as claimed in claim 1,
d) bioconverting the substrate to gamma-undecenolactone of formula (I),
e) recovering the gamma-undecenolactone of formula (I) produced.

7. The process as claimed in either one of claims 5 and 6, **characterized in that** said substrate is added in step c) as a mixture with at least one production auxiliary product, preferably chosen from oils, in particular sunflower oil that is hydrogenated or rich in oleic acid, mygliol or glucose, or a mixture of these ingredients.

8. The process as claimed in any one of claims 5 to 7, **characterized in that** step e) is an extraction of the gamma-undecenolactone by hydrodistillation, optionally followed by esterification and elimination of the substrate which has not reacted.

9. The process as claimed in any one of claims 5 to 7, **characterized in that** step e) is a solvent extraction of the gamma-undecenolactone obtained at the end of step d).

10. The process as claimed in any one of claims 5 to 7, **characterized in that** step e) is preceded by a step e') consisting of the in situ reduction of the undecenolactone obtained at the end of step d).

11. The process as claimed in claim 10, **characterized in that** the product derived from said step e') is an undecalactone.

12. A stereoselective process for preparing gamma-undecenolactone as claimed in any one of claims 5 to 10, **characterized in that** the microbial strain targeted in step a) is chosen from strains of the genus *Aspergillus sp.,* preferably *Aspergillus oryzae*, or from strains of the genus Mortierella sp., preferably *Mortierella isabellina.*

13. A perfumery composition, **characterized in that** it comprises an undecenolactone as defined in any one of claims 1 to 4.

14. A food composition, **characterized in that** it comprises an undecenolactone as defined in any one of claims 1 to 4.

15. A food additive, **characterized in that** it comprises an undecenolactone as defined in any one of claims 1 to 4.

16. The use of gamma-undecenolactone as defined in any one of claims 1 to 4 for the manufacture of perfumes, odorant substances or compositions, or cosmetic compositions, or as an odorant and/or sapid food additive.

17. The use of gamma-undecenolactone as defined in any one of claims 1 to 4, as a food flavoring agent.

18. The use of a culture of *Aspergillus oryzae* for the biosynthesis of (R)-gamma-undecenolactone as defined in claim 2.

19. The use of a culture of *Mortierella isabellina* for the biosynthesis of (S)-gamma-undecenolactone as defined in claim 3.

## Patentansprüche

1. Gamma-Undecenolacton der Formel (I): 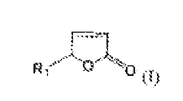 worin der Lactonring eine Ungesättigtheit zwischen dem Kohlenstoffatom Nr. 2 und dem Kohlenstoffatom Nr. 3 tragen kann und R1 eine C7-Alkenylgruppe, die eine einzige Alken-Ungesättigtheit an C10-C11 trägt, oder eine C7-Alkenylgruppe ist, die mehrere Ungesättigtheiten enthält, darunter eine Alken-Ungesättigtheit an C10-C11 und mindestens eine andere Alken-Ungesättigtheit an einem anderen Kohlenstoffatom als C7, wobei die Gruppe R1 gegebenenfalls substituiert ist.

2. Gamma-Undecenolacton nach Anspruch 1, wobei das asymmetrische Kohlenstoffatom an Position 4 in der (R)-Konfiguration vorliegt.

3. Gamma-Undecenolacton nach Anspruch 1, wobei das asymmetrische Kohlenstoffatom an Position 4 in der (S)-Konfiguration vorliegt.

4. Gamma-Undecenolacton nach einem der Ansprüche 1 bis 3, worin der Lactonring keine Ungesättigtheit trägt und R1 eine Gruppe C7H13, vorzugsweise CH2=CH-CH2-CH2-CH2-CH2-CH2-, ist.

5. Verfahren zur Herstellung eines gamma-Undecenolactons nach einem der Ansprüche 1 bis 4, **dadurch gekennzeichnet, dass** man eine mikrobielle Biosynthese des gamma-Lactons durchführt, ausgehend von mindestens einem Substrat, insbesondere Undecylensäure oder einem ihrer Ester, vorzugsweise Methyl- oder Ethylester, mithilfe einer mikrobiellen Kultur eines Stammes, der ausgewählt ist aus solchen, die eine Hydroxylierung des Substrats an C4 gestatten.

6. Verfahren nach Anspruch 5, **dadurch gekennzeichnet, dass** es folgende Schritte umfasst:
(a) Auswahl eines geeigneten Stammes, der ausgewählt ist aus solchen, die eine Hydroxylierung des Substrats an C4 gestatten,
(b) Kultur des besagten Stammes in einem geeigneten Kulturmedium, wobei der Kultur gegebenenfalls ein Schritt der Vorkultur des Stammes vorausgeht,
(c) Zugabe eines Substrates, das in gamma-Undecenolacton der Formel (I), wie im Anspruch 1 definiert, umgewandelt werden kann,
(d) biologische Umwandlung des Substrates in gamma-Undecenolacton der Formel (I),
(e) Gewinnung des produzierten gamma-Undecenolactons der Formel (I).

7. Verfahren nach einem der Ansprüche 5 oder 6, **dadurch gekennzeichnet, dass** das Substrat im Schritt c) im Gemisch mit mindestens einem Herstellungshilfsstoff zugegeben wird, der vorzugsweise ausgewählt ist aus Ölen, insbesondere hydriertem oder an Ölsäure reichem Sonneblumenöl, Mygliol oder Glucose oder einem Gemisch dieser Inhaltsstoffe.

8. Verfahren nach einem der Ansprüche 5 bis 7, **dadurch gekennzeichnet, dass** der Schritt c) eine Extraktion des gamma-Undecenolactons mittels Hydrodestillation, gegebenenfalls gefolgt von einer Veresterung und einer Beseitigung des nicht umgesetzten Substrats, ist.

9. Verfahren nach einem der Ansprüche 5 bis 7, **dadurch gekennzeichnet, dass** der Schritt e) eine Lösungsmittelextraktion des gamma-Undecenolactons ist, das am Ende von Schritt d) erhalten wird.

10. Verfahren nach einem der Ansprüche 5 bis 7, **dadurch gekennzeichnet, dass** dem Schritt e) ein Schritt e') vorausgeht, in dem das am Ende von Schritt d) erhaltene Undecenolacton in situ reduziert wird.

11. Verfahren nach Anspruch 10, **dadurch gekennzeichnet, dass** es sich bei dem aus diesem Schritt e') erhaltenen Produkt um ein Undecalacton handelt.

12. Stereoselektives Verfahren zur Herstellung von gamma-Undecenolacton nach einem der Ansprüche 5 bis 10, **dadurch gekennzeichnet, dass** der im Schritt a) eingesetzte Mikrobenstamm aus den Stämmen der Gattung Aspergillus sp., vorzugsweise Aspergillus oryzae, oder aus den Stämmen der Gattung Mortierella sp., vorzugsweise Mortierella isabellina, ausgewählt ist.

13. Parfümeriezusammensetzung, **dadurch gekennzeichnet, dass** sie ein Undecenolacton nach einem der Ansprüche 1 bis 4 umfasst.

14. Lebensmittelzusammensetzung, **dadurch gekennzeichnet, dass** sie ein Undecenolacton nach einem der Ansprüche 1 bis 4 umfasst.

15. Lebensmittelzusatz, **dadurch gekennzeichnet, dass** er ein Undecenolacton nach einem der Ansprüche 1 bis 4 umfasst.

16. Verwendung des gamma-Undecenolactons nach einem der Ansprüche 1 bis 4 zur Herstellung von Parfüms, Duftsubstanzen oder -zusammensetzungen, Kosmetikzusammensetzungen oder als duftender und/oder schmackhafter Lebensmittelzusatz.

17. Verwendung des gamma-Undecenolactons nach einem der Ansprüche 1 bis 4 zur Lebensmittelaromatik.

18. Verwendung einer Kultur von Aspergillus oryzae zur Biosynthese von (R)-gamma-Undecenolacton nach Anspruch 2.

19. Verwendung einer Kultur von Mortierella isabellina zur Biosynthese von (S)-gamma-Undecenolacton nach Anspruch 3.
